Europäisches Patentamt

(19) European Patent Office

Office européen des brevets

(11) EP 0 688 200 B1

(12) **FASCICULE DE BREVET EUROPEEN**

(45) Date de publication et mention
de la délivrance du brevet:
**07.04.1999 Bulletin 1999/14**

(21) Numéro de dépôt: 95902809.3

(22) Date de dépôt: 30.11.1994

(51) Int. Cl.$^6$: **A61F 13/15**

(86) Numéro de dépôt international:
**PCT/FR94/01391**

(87) Numéro de publication internationale:
**WO 95/15138 (08.06.1995 Gazette 1995/24)**

(54) **ARTICLE D'HYGIENE FEMININE MUNI DE CAPTEURS DU FLUX MENSTRUEL, SON PROCEDE DE FABRICATION**

HYGIENISCHER ARTIKEL FÜR FRAUEN MIT VORRICHTUNG ZUM SAMMELN VON FLÜSSIGKEIT UND VERFAHREN ZU DESSEN HERSTELLUNG

FEMININE HYGIENE ARTICLE HAVING MENSTRUAL FLOW COLLECTORS, AND METHOD FOR MAKING SAME

(84) Etats contractants désignés:
**BE DE GB IT NL**

(30) Priorité: **03.12.1993 FR 9314513**

(43) Date de publication de la demande:
**27.12.1995 Bulletin 1995/52**

(73) Titulaire: **FORT JAMES FRANCE
68320 Kunheim (FR)**

(72) Inventeurs:
• **NEVEU, Jean-Louis
F-27690 Lery (FR)**
• **CASTEL, Didier
F-92400 Courbevoie (FR)**
• **LEFEBVRE DU GROSRIEZ, Carol
F-78600 Maisons Laffitte (FR)**
• **KHUN, Pierre
F-67390 Marckolsheim (FR)**
• **LE PORT, Hervé
F-68170 Rixheim (FR)**

(74) Mandataire: **David, Daniel et al
Fort James France
Service Propriété Industrielle,
23 Boulevard Georges Clemenceau,
B.P. 321
92402 Courbevoie Cédex (FR)**

(56) Documents cités:
EP-A- 0 429 802                  EP-A- 0 525 676
WO-A-91/01766                  US-A- 3 420 234
US-A- 3 905 372                  US-A- 5 169 394

**Description**

L'invention concerne généralement un article d'hygiène féminine tel qu'une serviette périodique ou un protège-slip. Cet article d'hygiène a pour originalité de comporter au moins un capteur du flux menstruel, partiellement disposé en surface de l'article et destiné à assurer un contact permanent entre le corps et la partie absorbante de l'article d'hygiène.

Dans le domaine de l'hygiène féminine, les protections externes classiques actuellement sur le marché ont pour défaut rhédibitoire d'entraîner des fuites notamment latérales qui endommagent et salissent les sous-vêtements. En effet, une protection périodique classique fixée par adhésif sur un sous-vêtement n'est pas en contact permanent avec le corps de l'utilisatrice notamment lorsque celle-ci se déplace ou change de position.

Quand l'utilisatrice est en position allongée, les fuites surviennent à l'avant ou à l'arrière de la serviette, du fait du mauvais placement de la serviette qui se décale par rapport au corps de la femme ou encore du fait de la forme de la serviette qui est prévue de préférence pour une utilisation sensiblement horizontale.

Des enquêtes ont rapporté que quinze à cinquante pour cent des femmes portant des serviettes périodiques, se plaignaient de fuites. L'absence de fuite et l'absorption sont les principaux critères de choix des femmes pour leurs serviettes.

Pour tenter de surmonter ces problèmes d'étanchéité et plus particulièrement d'étanchéité latérale, des perfectionnements ont été apportés sur les serviettes périodiques de type classique, c'est-à-dire du type comprenant un matelas absorbant les fluides, une couche supérieure perméable située sur le matelas absorbant et enveloppant le cas échéant ce matelas, et une couche inférieure imperméable située sous le matelas absorbant.

Le brevet européen n° 91412 décrit une serviette comportant des moyens élastiques fixés sur les bordures latérales de la serviette afin de soulever celles-ci le long du matelas, de provoquer la contraction d'au moins la partie centrale de la bordure et de retenir ces bordures dans leurs positions élevées afin de former des barrières latérales contre les fuites latérales.

Le brevet européen n° 134086 a pour objet une serviette hygiénique munie de volets latéraux qui, lorsqu'ils sont repliés sous le sous-vêtement, forment des joints ou barrières d'étanchéité au niveau de leurs axes de flexibilité.

La demande de brevet européen n° 520884 révèle un article où des bourrelets latéraux sont formés sur au moins une partie de la zone d'entrejambe. Dans un mode préféré de réalisation, la feuille imperméable constituant la face inférieure du matelas absorbant, est repliée longitudinalement et forme une doublure imperméable du bourrelet, améliorant ainsi l'étanchéité latérale.

Par ailleurs, une recherche de confort et de maintien a conduit au choix de formes dites anatomiques, c'est-à-dire "en sablier" ou rétrécies à l'entrejambe, qui épousent davantage le corps de la femme et permettent un meilleur positionnement de la serviette.

Cependant, ces solutions, pour éliminer les fuites latérales, ne sont que partielles.

D'autres recherches ont été orientées sur des articles d'hygiène féminine consistant en une protection externe munie d'une zone centrale absorbante protubérante qui vient se placer entre les lèvres de la vulve. Ce type d'article tout en étant externe, présente une zone semi-interne assurant ainsi un contact permanent entre le corps de l'utilisatrice et la partie externe de la protection. Des exemples de produits sont illustrés par un grand nombre de brevets dont le brevet américain n° 4046147, le brevet européen n° 0162451 et le brevet français n° 2653328. Cependant, ce genre d'article non seulement est inconfortable mais provoque parfois une rétention excessive des fluides dans la protubérance c'est-à-dire la zone semi-interne de l'article.

On retrouve les problèmes rencontrés avec les protections internes du type tampon, c'est-à-dire l'absorption excessive et la saturation du tampon absorbant qui provoquent de manière réversible des fuites.

Enfin, un autre axe de recherche a été développé pour munir les articles d'hygiène d'une couche de transfert à base de fibres, destinée à améliorer le drainage des fluides.

Le brevet américain n° 3665922 révèle une serviette périodique qui, de par sa structure, reçoit immédiatement les fluides qui se déchargent, les transporte rapidement en les éloignant de leur point de décharge et les retient de manière efficace dans sa partie interne absorbante, tout en laissant la face externe supérieure de la serviette relativement sèche. De ce fait, les fuites latérales diminuent.

Plus précisément, ce brevet décrit une serviette périodique dont la couche de revêtement externe formant enveloppe comprend une multiplicité de fibres hydrophobes bouclées individuellement et constituant une couche de non-tissé de type "high loft". Chaque boucle est constituée d'une fibre monobrin. Les extrémités de chaque boucle sont fixées dans la base du non-tissé au moyen d'une couche adhésive.

Bien que cette couche superficielle de fibres améliore le transport et l'aspect sec de la surface de l'article située côté corps, les fibres d'un denier très faible : d'environ 1,5 à 3,0, et de surcroît de faible hauteur n'ont pas une résilience suffisante pour rester en contact permanent avec le corps de l'utilisatrice. Ces fibres peuvent même gêner à la limite la capture des fluides au niveau du corps. De plus, cette couche de fibres n'assure le transport que de manière localisée, les fibres ne traversent pas la structure interne de la serviette et ne pénètrent pas dans le matelas.

Le brevet américain n° 3967623 décrit généralement une couche de fibres et plus précisément de poils, qui se situe sur la face extérieure de la serviette hygiénique au-dessus d'un voile perforé. Cette couche peut être obtenue par fibrillation à chaud d'une feuille de polymères thermoplastiques.

Dans un mode de réalisation spécifique, ce matériau formant une couche de poils est présent non seulement à la surface de l'article comme couche de revêtement mais également comme couche interne entre le voile perforé et le matelas absorbant, les poils étant tournés vers le matelas. Les fibres individuelles de cette couche interne s'entremêlent avec les fibres individuelles du matelas et créent des canaux dans le matelas où les fluides circulent.

Ainsi, la couche interne dont les fibres ont été traitées par un agent tensio- actif et devenant ainsi hydrophiles en surface, a pour fonction de transporter les fluides vers le matelas.

De même ici, les couches de fibres ou poils n'assurent le transport des fluides que de manière localisée. Les fibres ne sont pas organisées entre elles, ni ne traversent l'article d'hygiène de sa face supérieure côté corps jusqu'à sa face inférieure côté sous-vêtement. De plus, dans le cas d'une décharge importante de fluides, la couche superficielle de fibres ou poils est si dense que les fluides vont avoir tendance à s'écouler sur la surface des poils sans pouvoir pénétrer dans ce revêtement, ce qui peut provoquer en conséquence des fuites latérales.

La demande de brevet internationale WO 93/01780 décrit de manière générale une serviette périodique comprenant un voile de surface perméable aux liquides, une couche inférieure imperméable aux liquides, un matelas absorbant positionné entre le voile et la couche inférieure et une couche de transport comportant des éléments orientés dans une direction Z perpendiculaire au plan formé par la serviette. La partie inférieure de cette couche de transport peut être insérée dans le matelas et la partie supérieure de cette couche peut s'étendre au-dessus de la surface du matelas.

Les éléments constituant cette couche de transport sont des fibres formant capillaires. Plus précisément, elles ont une forme et plus précisément une section telle, que des canaux se créent sur leur surface externe à l'intérieur d'une même fibre. Ces fibres ont par exemple une section en U ou en H, les canaux étant formés par exemple par l'espace entre les jambes du H. La capillarité à l'intérieur de la couche de transport est assurée par chaque fibre. On ne décrit pas d'arrangement précis des fibres entre elles si ce n'est l'assemblage en touffes qui reste un assemblage au hasard sans positionnement des fibres les unes par rapport aux autres. Les fibres formant capillaires ont un denier variant dans l'intervalle de 10 à 35. Elles sont monobrins.

De manière générale, la couche de transport comportant ces fibres, est disposée sous le voile de surface perméable. Dans deux modes de réalisation précis, certains éléments de la couche de transport dépassent à la surface du voile. Dans un premier cas, certaines fibres passent à travers les perforations du voile et leurs extrémités apparaissent en surface. Mais compte-tenu de la petite taille des fibres, la hauteur de dépassement des fibres au-dessus du voile est très réduite et le contact des fibres avec le corps ne peut être assuré de manière permanente.

Dans un second cas, une fenêtre est découpée dans le voile si bien qu'une partie des fibres est découverte en surface de la serviette périodique. Cependant, il est vraisemblable que ces fibres n'ont pas non plus dans ce cas, une hauteur suffisante, ni une résilience suffisante pour être au contact permanent de la source d'écoulement des fluides, au niveau du corps.

Par ailleurs, dans tous les modes de réalisation décrits dans cette demande, le gradient de capillarité est assuré pour ce dispositif formant serviette périodique, par un ensemble de plusieurs matériaux distincts, de la surface vers le fond de l'article d'hygiène :

- les perforations du voile, d'un certain diamètre,
- les canaux capillaires intrafibres des fibres monobrins constituant la couche de transport, de diamètre plus faible, et
- les canaux interfibres des fibres du matelas absorbant, de diamètre encore plus faible,

Ce dispositif permet de drainer les fluides déposés à la surface du voile en laissant ce voile sec et de transporter les fluides vers le matelas dans des zones absorbantes insaturées. Mais, il ne peut assurer efficacement à la fois la capture des fluides à leur point d'écoulement directement au contact du corps et le drainage rapide par un même matériau assurant la capillarité, de ce point d'écoulement jusqu'au fond du matelas.

Les fibres ne sont pas organisées entre elles pour créer un capteur assurant lui-même le transport des fluides à travers toute la structure interne de la serviette jusqu'au fond du matelas.

De ce fait, des opérations supplémentaires de fabrication sont nécessaires pour renforcer le contact de la couche de transport, et donc des fibres, d'une part avec le voile perforé et d'autre part, avec le matelas absorbant.

Par ailleurs, la couche de transport fait partie intégrante de la structure interne de l'article d'hygiène. Dans un mode de réalisation comportant les touffes disposées sous le voile, celles-ci sont formées en prélevant de la couche de transport située à l'intérieur du matelas absorbant, de la bourre, à travers une fente réalisée en surface du matelas. Dans un autre mode de réalisation, chaque fibre formant capillaire est cousue dans le matelas pour former une bouclette, et le voile est ensuite placé sur le pré-assemblage par tout moyen mécanique tel que par dépôt d'adhésif et le cas échéant est découpé. A l'intérieur d'une bouclette, la capillarité n'est assurée que par la fibre en elle-même.

Ces articles d'hygiène munis d'une couche fibreuse de transport sont un net progrès dans l'amélioration du drainage des fluides menstruels sans pour autant pouvoir assurer de manière certaine la capture des fluides à leur source d'écoulement.

A partir de l'observation du phénomène des fuites latérales, on a pu établir les causes essentielles des fuites en général :

- l'absence de contact permanent entre le corps et la protection externe absorbante, ceci notamment lorsque l'utilisatrice est en position allongée,
- la diversité des morphologies d'entrejambe,
- la présence de poils pubiens qui peuvent drainer les fluides menstruels vers les bordures latérales de la protection voire à l'extérieur de la protection,
- le manque d'absorption dû à la nature et à l'abondance des écoulements, et
- la déformation de la protection externe au porter, qui a subi des contraintes et a été humidifiée.

Les inventeurs ont eu l'idée de munir la protection externe de type classique d'un ou plusieurs capteurs qui, à l'image des poils pubiens, captent les fluides à leur point d'écoulement mais à l'inverse, les drainent de manière guidée jusqu'au fond du matelas de la protection. Ces capteurs assurent le relais entre le corps et la protection absorbante et optimisent la vitesse de pénétration des fluides dans la protection absorbante.

L'invention a pour but de pallier l'ensemble des inconvénients mentionnés précédemment en réduisant le taux de fuite sensiblement à 0 %

Pour ce faire, l'invention a pour but d'assurer un contact permanent entre le corps et la protection absorbante de type classique, quels que soient la position et les mouvements de l'utilisatrice.

L'invention a pour but non seulement de capter mais également de drainer de gros débits d'écoulement de fluides, les capteurs ayant une fonction "temporaire" de réservoir.

L'invention a aussi pour but de fournir un article d'hygiène dont la partie des capteurs située en surface de l'article d'hygiène, est résiliente, résistante à l'écrasement et s'adaptant aux différentes morphologies d'entrejambe.

L'invention a encore pour but de fournir un article d'hygiène muni de capteurs dont le diamètre apparent (celui de la partie du capteur située en surface de l'article d'hygiène), est de l'ordre du diamètre d'un poil pubien.

L'invention a en outre pour but de réduire considérablement la taille des serviettes hygiéniques en les rendant très discrètes, en proposant pour une serviette de grosse capacité d'absorption, la taille d'un protège-slip

L'invention a encore pour but de fournir un article d'hygiène féminine de structure très simple et réalisable industriellement

L'invention a également pour but de pouvoir supprimer le cas échéant la présence d'un voile perforé à la surface de l'article d'hygiène.

L'invention a de plus pour but de fournir un procédé simple de fabrication d'une serviette munie de capteurs à partir d'un article d'hygiène féminine pré-assemblé.

L'article d'hygiène féminine selon l'invention est du type comprenant un matelas absorbant les fluides, une couche de surface située sur ledit matelas absorbant et une couche imperméable située sous ledit matelas et destinée à être au contact du sous-vêtement.

Suivant une caractéristique essentielle de l'invention, l'article d'hygiène comporte au moins un capteur de fluides dont l'extrémité libre supérieure est à une distance d de ladite couche de surface et est destinée à être au contact du corps et dont l'extrémité inférieure est située en-dessous de la face inférieure dudit matelas, et en ce que ledit capteur est formé d'un faisceau comprenant au moins un élément fibreux multibrins, la partie dudit capteur ou faisceau située au-dessus de ladite couche de surface ayant une forme évasée convergente.

Selon une caractéristique avantageuse de l'invention, ledit capteur est muni de canaux capillaires souples et permanents formés par les espaces entre les brins dudit faisceau constituant le capteur.

Selon une autre caractéristique avantageuse de l'invention, ledit faisceau est une bouclette ouverte ou fermée comprenant au moins un fil multibrins.

L'expression "bouclette ouverte" correspond ici et dans le texte qui suit, à une bouclette qui a été rasée ou coupée en son extrémité, ce qui conduit à un faisceau ou une "touffe" de type velours.

L'invention a également pour objet un procédé pour fabriquer un tel article d'hygiène.

Selon une des caractéristiques essentielles du procédé selon l'invention, il consiste à :

a) former un pré-assemblage du type comprenant un matelas absorbant les fluides et une couche de surface disposée sur ledit matelas ;
b) disposer une couche externe fibreuse sous ou sur ledit pré-assemblage ;
c) former à partir de ladite couche fibreuse, au moins un faisceau comprenant au moins un élément fibreux multibrins dont l'extrémité inférieure est fixée au niveau de la face inférieure du matelas, dont la partie externe située au-dessus de ladite couche de surface est libre et de forme évasée convergente vers la couche de surface précitée et dont l'extrémité supérieure est destinée à être au contact du corps ; et

d) à fixer une couche imperméable sous l'article d'hygiène obtenu en c).

D'autres caractéristiques et avantages de l'invention apparaîtront plus clairement à la lecture de la description détaillée de l'invention qui suit, en référence aux dessins dans lesquels :

- la figure 1 représente une vue de dessus de l'article d'hygiène selon l'invention, suivant un mode de réalisation ;
- la figure 2 représente une coupe transversale de l'article représenté en figure 1, suivant l'axe II-II ;
- la figure 3 représente le détail d'un capteur représenté en figure 2 ;
- la figure 4a est une photographie d'un capteur sous forme de bouclette et plus précisément de la partie du capteur ou bouclette située juste au-dessus de la couche de surface ;
- la figure 4b est une photographie du capteur de la figure 4a et illustre le drainage des fluides au sein de ce capteur ;
- la figure 5 représente une coupe transversale d'un autre mode de réalisation selon l'invention où les bouclettes formant les capteurs sont ouvertes ;
- les figures 6a à 6e sont des photographies d'une bouclette ouverte formant capteur, illustrant la capture et le drainage d'une goutte de fluide dans l'article d'hygiène absorbant ;
- la figure 7 représente la surface d'un article d'hygiène recouverte de bouclettes dont la hauteur est adaptée à la morphologie d'un entrejambe ;
- les figures 8a, 8b et 8c représentent la coupe de l'article représenté en figure 7 suivant respectivement les axes VIIIa-VIIIa, VIIIb-VIIIb et VIIIc-VIIIc ;
- les figures 9a, 9b et 9c illustrent les différentes étapes d'un procédé de rasage suivant une forme ;
- la figure 10 représente une coupe longitudinale de l'article représenté en figure 1, suivant l'axe IX-IX, et
- la figure 11 représente une courbe du temps d'acquisition en liquide d'un article d'hygiène selon l'invention, en fonction du nombre de capteurs prévus sur cet article.

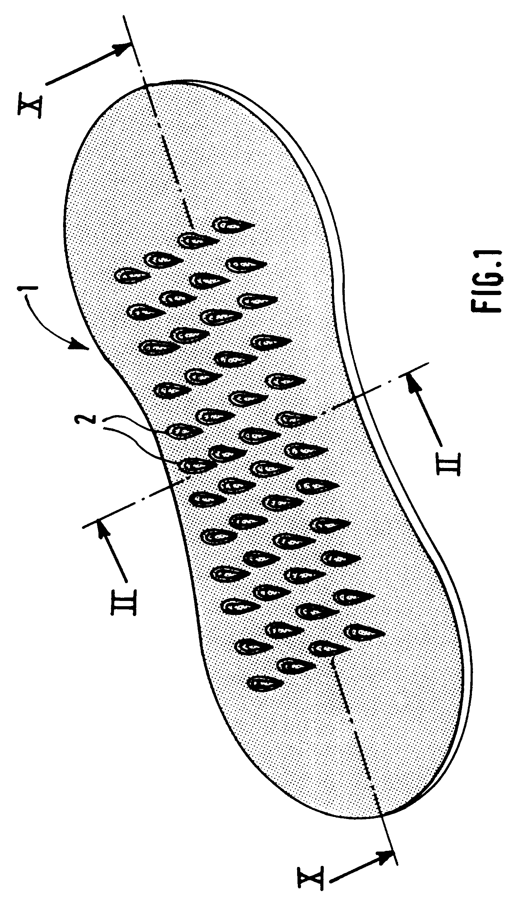

En référence aux figures 1 à 3, l'article d'hygiène selon l'invention est une protection hygiénique 1 externe de type classique qui comporte avantageusement au moins un moyen formant capteur 2 assurant d'une part, la capture des fluides à leur point d'écoulement au niveau du corps, c'est-à-dire à un endroit situé au contact du corps le plus près possible de la source d'écoulement du flux menstruel, et d'autre part, le drainage des fluides de ce point de contact avec le corps jusqu'à la structure interne absorbante de la protection.

De manière générale, la protection externe 1 comprend un matelas absorbant 3, une couche de surface 4 située sur le matelas absorbant 3 et une couche imperméable 5 située sous le matelas absorbant et destinée à être au contact du sous-vêtement par tout moyen classique de fixation tel qu'un adhésif.

De préférence, avant utilisation, cette protection est munie d'un moyen de protection 6 du moyen de fixation. Dans le cas de la fixation par adhésif, ce moyen peut être une bande siliconée.

Le moyen formant capteur 2 est constitué d'un faisceau comprenant au moins un élément fibreux multibrins, dont la partie externe 7 est située au-dessus de la couche de surface 4 et dont la partie interne 8 est située sous cette couche de surface et traverse le matelas absorbant. La partie externe 7 est libre et de forme évasée et convergente vers la couche de surface 4. Cette partie externe peut prendre une forme sensiblement tronconique ou de manière plus imagée "d'entonnoir". Plus précisément, les éléments fibreux multibrins sont assemblés à l'intérieur du faisceau de manière à créer un "réceptable" de forme évasée dont la base est resserrée au niveau de la surface de l'article d'hygiène, c'est-à-dire de la couche de surface. L'extrémité libre supérieure 9 du capteur 2 est destinée à venir au contact du corps et à capter les fluides.

La partie externe est souple, résiliente, et a une hauteur d de l'ordre de 3 à 20 millimètres, de préférence de l'ordre de 5 à 10 millimètres, de manière que l'extrémité supérieure 9 du capteur soit en contact permanent avec le corps quels que soient la position de l'utilisatrice et les mouvements et déplacements du corps par rapport à la protection externe fixée sur le sous-vêtement.

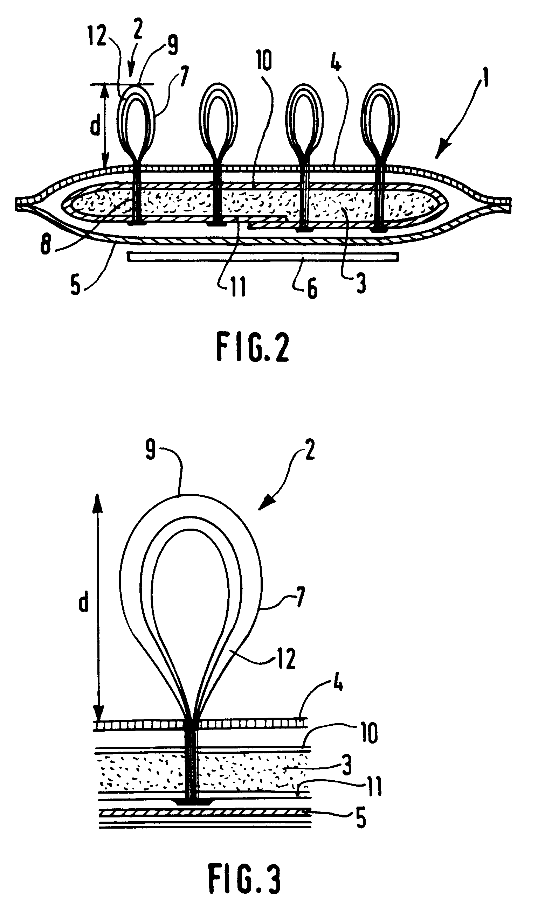

Dans la partie interne 8 du faisceau formant le capteur, les éléments fibreux sont plus resserrés entre eux et sont organisés sous forme de fuseaux avec des zones de resserrement correspondant au passage de chaque couche de matériau telle que la couche de surface ou les couches formant l'enveloppe du matelas, représentées sur la figure 2 par l'enveloppe supérieure 10 et inférieure 11 du matelas 3.

Le capteur a pour spécificité de créer un réseau capillaire à l'intérieur du faisceau. En effet, l'organisation des brins à l'intérieur de ce faisceau forme des canaux capillaires interbrins 12, ces canaux étant permanents et souples.

Le gradient de capillarité est assuré par le capteur lui-même. En effet, le diamètre des canaux décroît de l'extrémité supérieure du capteur vers la base de la partie externe et convergente du capteur située au niveau de la couche de surface où les brins sont resserrés au passage de cette couche, le diamètre des canaux ou espaces interbrins continue de décroître au niveau de chaque matériau traversé, jusqu'au fond du matelas, et plus particulièrement de l'enveloppe inférieure 11 du matelas où le diamètre des canaux est le plus faible.

La présente invention assure donc la capillarité au sein d'un même matériau formé d'éléments fibreux multibrins et dans une même unité ou faisceau d'éléments fibreux organisés formant un capteur, à travers toute la structure d'un article d'hygiène.

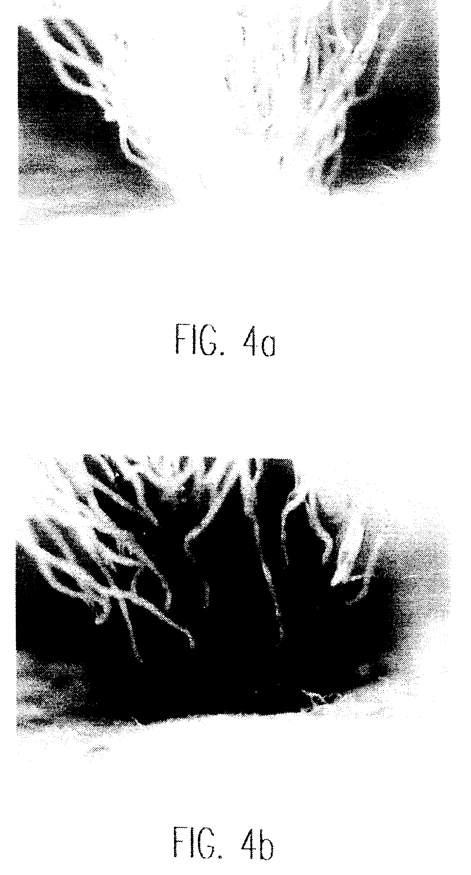

Ces capteurs ont pour avantage de par leur nature, de pouvoir capter et contenir de manière transitoire une importante quantité de fluides lors de décharges du flux menstruel, sous des débits élevés, et forme ainsi un réservoir temporaire qui draine simultanément et directement les fluides dans la partie absorbante de la serviette. Cette capacité à la fois de réservoir et de drainage est bien illustrée par les figures 4a et 4b où la figure 4b est une photographie de la partie externe d'un capteur en fonctionnement, montrant la capture et le guidage des fluides dans le réseau capillaire formé par les brins du faisceau.

Ces capteurs ont également pour avantage, en ce qui concerne leur partie externe d'être résilients et résistants a l'écrasement. Ils reprennent leur forme et leur position après déformation, par exemple lors des mouvements ou changements de position de l'utilisatrice. Le choix de matériaux fibreux appropriés présentant de telles caractéristiques sera développé dans la description qui suit

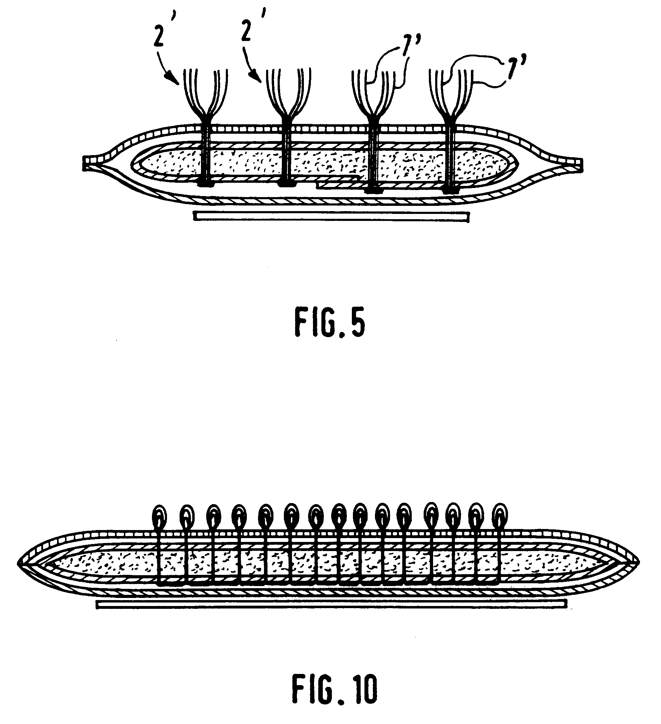

Les faisceaux peuvent se présenter sous la forme de bouclettes ouvertes ou fermées. Les bouclettes sont ouvertes lorsqu'elles sont coupées ou rasées en leur extrémité. Ce mode de réalisation est représenté en figure 5, par la partie externe 7' des capteurs 2'.

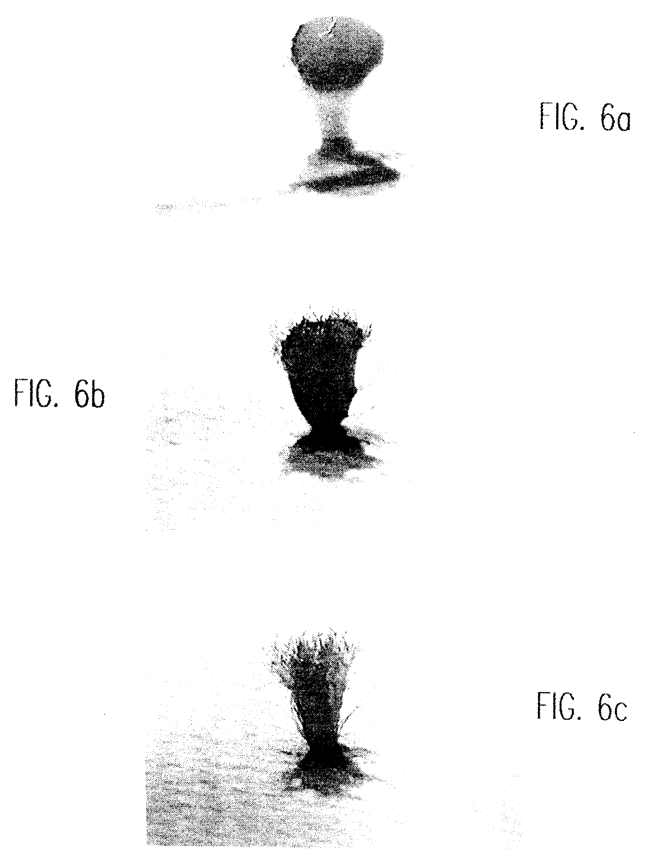

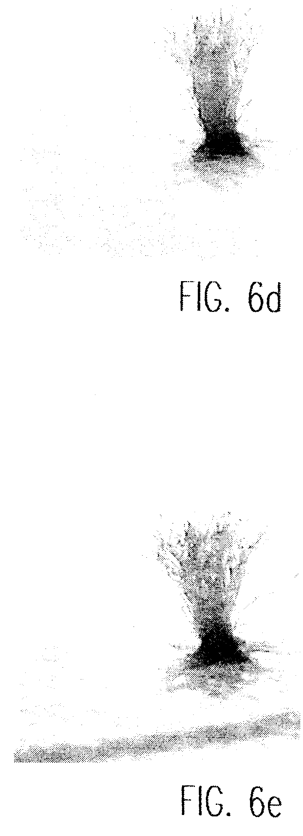

Les figures 6a à 6e sont des photographies d'un capteur sous forme de bouclette dont le sommet a été rasé. Ces figures illustrent la capture d'une goutte de fluide au sommet de la bouclette (figure 6a) et le drainage des fluides de cette extrémité supérieure du capteur jusqu'à la base de sa partie externe (figure 6d et 6e).

Comme on peut l'observer, le faisceau formé d'éléments fibreux multibrins se vide au fur et à mesure de la descente du fluide par capillarité dans la partie absorbante de la serviette. Il est à noter que la couche de surface reste sèche, le fluide n'entrant quasiment pas en contact des matériaux formant la couche de surface. La forme évasée convergente combinée aux espaces interbrins formant le réseau capillaire, favorise avantageusement le drainage rapide des fluides. Cette série de photographies illustre également la bonne cohésion des brins à l'intérieur du faisceau.

La distribution des capteurs à la surface de l'article est un autre paramètre améliorant le produit.

Les capteurs tels que des bouclettes peuvent être localisés au centre de l'article de manière à être concentrés dans une zone où ils seront au contact de la partie du corps où se situe la source d'écoulement des fluides.

Ces capteurs peuvent être disposés longitudinalement à l'article, en plusieurs rangées et être plus ou moins resserrés transversalement. Ces rangées peuvent être plus ou moins larges. Les capteurs peuvent être disposés suivant un motif ovale ou autre sur la surface de l'article d'hygiène Les capteurs peuvent par exemple être disposés dans la partie centrale de l'article suivant des cercles concentriques avec des capteurs de hauteur maximale située au centre Dans un mode de réalisation particulier, la surface de l'article d'hygiène ou la couche de surface peut être totalement recouverte de capteurs. De manière avantageuse, la hauteur des capteurs varie en fonction de la morphologie d'entrejambes.

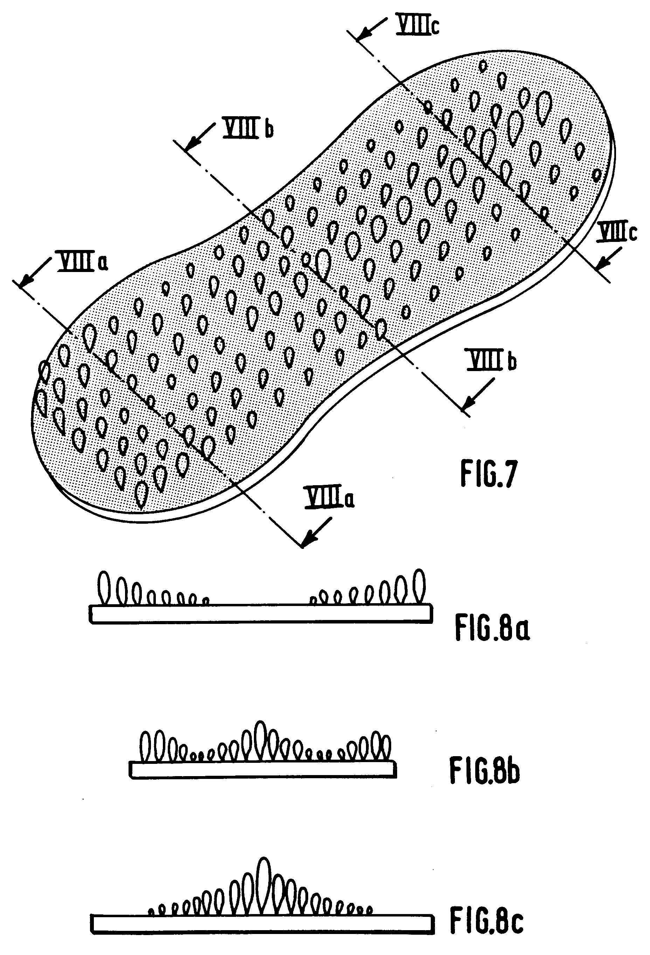

Pour ce faire, des capteurs de hauteur variable sont prévus. Ceci est illustré par la figure 7 représentant schématiquement la surface d'une serviette où les capteurs sont des bouclettes. Les figures 8a, 8b et 8c montrent la répartition des hauteurs de bouclettes respectivement pour la partie avant, la partie centrale et la partie arrière de la serviette périodique représentée en figure 7, correspondant aux profils des parties avant, centrale et arrière de la région pubienne.

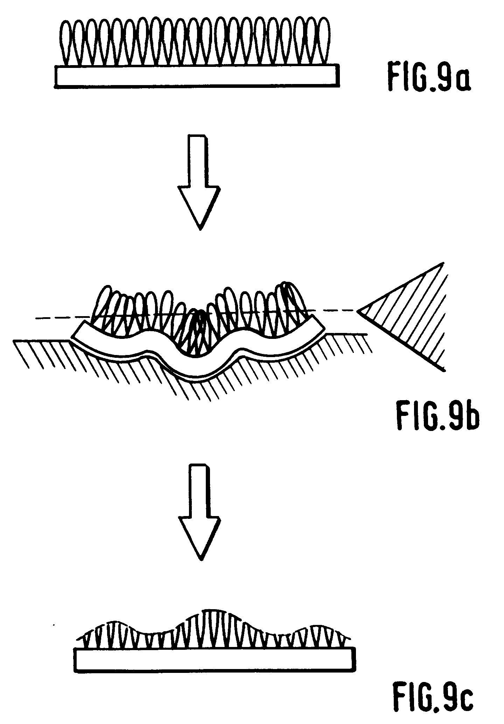

Un profil de cette région est réalisable par moulage. Ainsi, à partir du contre-moulage ou contre-forme, un motif peut être reproduit pour la répartition des capteurs de hauteur différente à la surface de l'article d'hygiène. Dans ce cas, la surface totale de l'article est recouverte de bouclettes d'une hauteur maximale donnée, l'article est appliqué contre un moulage correspondant à la zone d'entrejambes et les bouclettes sont ensuite rasées de manière à reproduire le profil de l'entrejambe. Les figures 9a, 9b et 9c illustrent les différentes étapes de ce procédé de rasage suivant une forme.

Une conséquence avantageuse des capteurs sur l'article d'hygiène selon l'invention, est l'augmentation non négligeable de la capacité d'absorption de la serviette. De ce fait, il est possible de réduire considérablement la taille de la serviette en choisissant des matériaux absorbants appropriés. En effet, cette dernière pour une capacité d'absorption d'une serviette de la gamme "normale" peut prendre la taille d'un protège-slip, c'est-à-dire d'une part, être très mince et d'autre part, de longueur réduite de manière à seulement couvrir sensiblement la région pubienne.

Par ailleurs, l'article d'hygiène selon l'invention peut comporter le cas échéant des volets latéraux. Mais le rôle de ces derniers en tant que barrière latérale n'est plus indispensable.

Un autre avantage de l'article selon l'invention est la possibilité d'utiliser tout type de matériau pour la couche de surface, c'est-à-dire un matériau plus ou moins perméable, la pénétration des fluides étant assurée par les capteurs. Un article dont la couche de surface serait un matériau imperméable, serait tout à fait concevable.On pourrait envisager tout matériau présentant la propriété d'être perméable à l'air ou à la vapeur d'eau tout en étant imperméable aux liquides. La perméabilité aux liquides n'est donc plus un critère absolument nécessaire pour cette couche de surface. Ainsi, d'autres matériaux que les voiles plastiques perforés de plus en plus sophistiqués et coûteux, peuvent être utilisés De simples matériaux assurant le maintien du matelas sont suffisants. A ce titre, un article d'hygiène où par exemple la couche de surface est confondue avec l'enveloppe du matelas, fait partie de l'invention. Un matériau textile non tissé, tissé ou tricoté, de faible grammage, est également approprié. La couche de surface peut être formée d'un mélange de fibres hydrophiles et hydrophobes.

De préférence, cette couche de surface est un matériau non tissé, cardé ou non, tel qu'un nontissé "highloft" ou un matériau de type "meltblown" ou encore un matériau formé de filaments continus thermoliés (technologie "spun-laid").

De manière générale, les matériaux classiquement utilisés pour les serviettes minces sont tout à fait transposables au produit selon la présente invention, excepté la couche de surface dont la perméabilité optimale n'est plus recherchée.

Le matelas absorbant peut être constitué de tout matériau absorbant classique. Dans un mode de réalisation préféré, ce matelas est formé de fibres cellulosiques contenant une matière superabsorbante sous forme de poudre. Par exemple, une bande de fibres cellulosiques, papetières, formée par voie sèche et liée par un latex, c'est-à-dire un matériau de type "air-laid", dont les deux bords latéraux sont rabattus sur une partie centrale sur laquelle est déposée au préalable le matériau superabsorbant, peut former ce type de matelas. Un tel matelas est représenté en figure 2. Le matériau superabsorbant peut être sous forme de poudre, de fibres, de feuille et également de poudre liée à des fibres synthétiques. On peut également envisager un matelas constitué d'un mélange de fibres cellulosiques et de fibres synthétiques, placé entre deux couches d'ouate de cellulose, l'ensemble étant thermolié.

D'autres matériaux tels qu'une mousse synthétique, par exemple de polyuréthane, ou naturelle, par exemple de sphaigne traitée, présentant de grandes capacités d'absorption, peuvent être utilisés. Le traitement de la sphaigne mentionnée ci-dessus est décrit dans la demande de brevet européen n° 0546585.

La couche imperméable située sous le matelas absorbant est en matériau plastique imperméable, tel que le polyéthylène. Elle peut également être constituée d'un matériau étant à la fois perméable à l'air et à la vapeur d'eau et imperméable aux liquides. Un tel matériau est par exemple un non-tissé de type "meltblown" composé de microfibres, par exemple de polyéthylène.

La couche de surface et la couche imperméable sont associées par exemple par scellage pour envelopper le matelas.

Le matériau formant les capteurs est maintenant décrit plus en détails.

L'élément fibreux multibrins formant le faisceau, est un filament ou un fil multibrins ou toute association de fibres en plusieurs brins. L'association d'au moins trois fibres pour former un élément fibreux lui-même sous forme de faisceau, fait partie de l'invention.

Ainsi, un capteur sous forme de bouclette ouverte ou fermée peut être constitué d'un à plusieurs filaments ou fils multibrins

Un élément fibreux tel qu'un fil est de préférence frisé ou texturisé, ce qui améliore sa résilience.

Chaque élément fibreux comprend entre environ 2 et environ 200 brins. Et chaque brin a un titre d'au moins 6dtex

De plus, cet élément fibreux a un titre d'au moins 50 dtex et se situe de préférence dans l'intervalle variant entre environ 180 et environ 5000 dtex.

Des éléments fibreux situés dans cette gamme de titre plus élevé sont appropriés pour les caractéristiques de résilience et de résistance à l'écrasement recherchées pour la partie externe des capteurs.

De préférence, chaque élément fibreux comprend entre environ 30 et environ 100 brins, le titre unitaire d'un brin étant d'au moins 17dtex.

Le choix du titre unitaire d'un brin et du nombre de brins par élément fibreux est fondamental pour obtenir le compromis entre la résilience et la douceur. Par exemple, pour un brin de titre faible, un grand nombre de brins sera nécessaire pour compenser la faible résilience.

L'élément fibreux en tant que tel est de préférence hydrophile mais n'est pas absorbant. Il peut être traité, par exemple par un agent tensioactif pour le rendre hydrophile.

L'élément fibreux peut être à base de fibres naturelles, artificielles, synthétiques, ou leurs mélanges. Les critères de choix de la nature de l'élément fibreux sont à la fois la résilience et la douceur.

Or, compte-tenu du titre élevé des éléments fibreux utilisés, il est nécessaire que ces derniers soient relativement doux au contact de la peau.

Dans les fibres naturelles, le coton ou le lin peut être utilisé, mais il doit être traité de manière à diminuer son hydrophilie. Dans le cas d'un élément fibreux en coton, il s'agit de fils retords, c'est-à-dire de deux ou trois fils associés entre eux par torsion.

Dans les fibres artificielles, la viscose est également appropriée si elle est traitée de manière à être moins hydrophile.

Enfin, les fibres synthétiques sont préférées et sont choisies parmi le polyester, le polyamide, le polyéthylène ou le polypropylène. On choisira plus préférentiellement des éléments fibreux en polyamide pour une plus grande douceur, ou en certains polyesters à la fois résilients et doux. Le polypropylène sera choisi si une très grande résilience est recherchée avant tout.

A l'intérieur d'un même faisceau, on peut combiner différentes natures fibreuses selon les propriétés recherchées : douceur, hydrophilie, et résilience. La hauteur des brins à l'intérieur d'un même faisceau peut aussi varier. La nature des éléments fibreux peut également changer d'un faisceau à l'autre ainsi que le nombre de brins par élément fibreux. On peut ainsi former des zones de capteurs, plus douces, et d'autres, plus résilientes. Il est aussi possible de créer par exemple des variations d'hydrophilie d'un capteur à l'autre ou d'une partie de la surface de l'article muni de capteurs à une autre partie de cette surface.

Différents modes de réalisation de l'article selon l'invention sont donnés ci-après, mais ne sont en aucun cas limitatifs.

Le procédé selon l'invention consiste à former des capteurs à partir d'une couche fibreuse située à l'extérieur d'un pré-assemblage, en les insérant partiellement dans ce pré-assemblage composé de différentes couches de matériaux formant l'article d'hygiène. Le capteur est ainsi formé par un apport de matériau extérieur dans un pré-assemblage existant.

Dans un premier temps, on forme un pré-assemblage en préparant un produit d'hygiène par exemple de type stratifié en posant une couche de surface sur un matelas absorbant. Puis on dispose sur ou sous ce pré-assemblage, une couche externe fibreuse. A partir de cette couche fibreuse, on forme au moins un capteur sous la forme d'un faisceau comprenant au moins un élément multibrins. L'extrémité inférieure de ce faisceau est fixée au niveau de la face inférieure du matelas et la partie externe de ce faisceau est libre au-dessus de la couche de surface et de forme évasée, convergente vers la couche de surface. Le faisceau traverse donc le pré-assemblage.

On fixe ensuite une couche imperméable sous le pré-assemblage muni de capteurs, par exemple par scellage des bordures de cette couche avec celles de la couche de surface dépassant du matelas, ou par tout autre moyen. Des moyens de fixation tels que des bandes d'adhésifs sont placés sous la couche imperméable. Enfin, des moyens de protection des moyens de fixation sont prévus, comme une bande siliconée placée sur les dépôts de bandes d'adhésifs.

Dans l'étape de fabrication des capteurs, plusieurs modes de réalisation sont possibles dont certains sont développés ci-après à titre illustratif

A partir d'une couche fibreuse externe prévue au-dessus du pré-assemblage et formée d'un fil multibrins continu ou de plusieurs fils multibrins discontinus, on peut piquer ce fil par couture à travers le pré-assemblage jusqu'au fond du matelas et former en surface une bouclette comprenant au moins un fil multibrins.

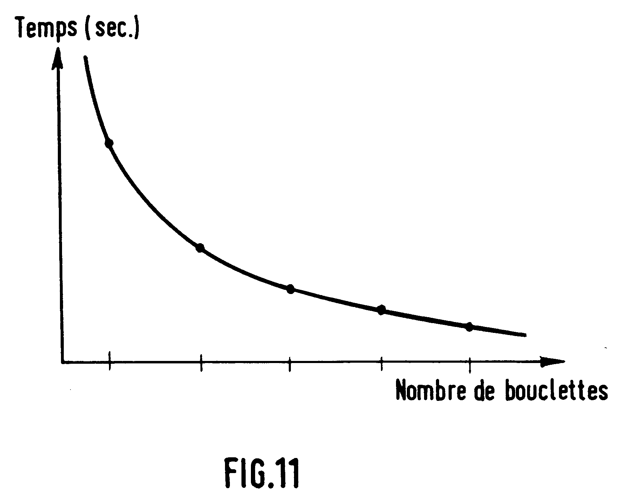

Le fil multibrins traverse chaque couche de matériau formant le pré-assemblage en un seul et même point. Un même fil continu peut servir à former toutes les bouclettes. On peut voir sur la figure 10 que les bouclettes d'une même rangée, obtenues par couture à partir d'un même fil continu, sont reliées les unes aux autres au niveau de la face inférieure du matelas, et plus précisément sous celle-ci.

Par conséquent, le fil court sous le matelas et diffuse les liquides d'un bout à l'autre de ce dernier.

Les bouclettes peuvent être directement cousues en plusieurs rangées.

Ces bouclettes formées par couture sont réalisables au moyen d'une machine à coudre, ou d'une machine du type Malipol qui forme des points de chaînette en traversant le pré-assemblage. Les bouclettes, une fois formées sont soit laissées intactes, soit coupées ou rasées. Le rasage des bouclettes peut être réalisé suivant une forme adaptée à l'entrejambe comme on l'a décrit précédemment.

La couche fibreuse correspondant à un ou plusieurs fils, est retirée une fois les capteurs formés.

Dans un autre mode de réalisation, la couche externe fibreuse est située sous le pré-assemblage et est également constituée d'au moins un fil multibrins continu ou discontinu.

Les capteurs sont fabriqués par une technique connue dite de tuftage, qui consiste à piquer au moyen d'une aiguille le fil à travers le pré-assemblage, à former une bouclette en faisant dépasser l'aiguille d'une hauteur d au-dessus de la couche de surface et en retirant cette aiguille. La bouclette peut être éventuellement coupée. On peut utiliser une machine de type monoaiguille à action unilatérale pour obtenir un petit nombre de bouclettes.

Dans ce type de machine, l'aiguille est munie d'une fourchette. Lorsque l'aiguille est piquée à travers le pré-assemblage, le fil reste pris par la fourchette après le retrait de l'aiguille et dépasse d'une hauteur d de la couche de surface. On travaille ici sur l'envers du pré-assemblage.

Des machines multiaiguilles appropriées pour traverser les différents matériaux du pré-assemblage sont également utilisables. Dans ce type de machine, plusieurs aiguilles sont prévues, mais afin d'obtenir des bouclettes suffisamment espacées les unes des autres, des machines ayant des aiguilles au moins tous les 2,5 mm dans le sens travers et tous les 2 mm dans le sens machine sont préférées.

Le choix de la couche de surface est dans ce cas également important, il doit être suffisamment résistant sous l'action des aiguilles. Des matériaux textiles non tissés tels que des nontissés formés de filaments continus thermoliés (technologie spun-laid) ou des nontissés de type meltblown, sont appropriés.

On peut également utiliser une feuille de plastique pour cette couche de surface.

Dans un autre mode de réalisation, la couche externe fibreuse est également disposée sous le pré-assemblage et plus précisément sous le matelas absorbant. Ici, cette couche fibreuse est une nappe de fibres, cardée. Dans ce cas, elle est associée au pré-assemblage et restera dans l'article d'hygiène fini.

Dans ce mode de réalisation, les capteurs peuvent être formés par une technique d'aiguilletage. La couche fibreuse qui est une nappe cardée, liée ou non, ou un voile, comprend des fibres de différentes longueurs, et de différents titres, frisées ou non frisées. Des éléments fibreux de la nappe sont ainsi introduits au moyen notamment d'aiguilles à travers le pré-assemblage et forment des faisceaux d'éléments fibreux multibrins correspondant aux capteurs dont une partie apparaît en surface du pré-assemblage, au-dessus de la couche de surface. La base de ces faisceaux est maintenue, le cas échéant, sous la nappe fibreuse par exemple par une couche d'adhésif ou par thermoliaison.

L'aiguilletage est de type classique, mécanique, c'est-à-dire réalisé au moyen d'aiguilles en acier. Les aiguilles sont munies de barbes.

Un tel procédé utilisant la technique d'aiguillage est tout à fait réalisable à l'échelle industrielle.

Un exemple d'article dont les capteurs sont formés par aiguilletage mécanique est donné ci-après.

Le pré-assemblage est constitué d'une nappe fibreuse cardée d'un grammage de 50 g/m$^2$ à base de fibres de polyéthylène, formant la couche fibreuse externe, recouverte d'une couche de fibres cellulosiques papetières liées par un latex et obtenue par voie sèche, ("air-laid"), d'un grammage de 65 g/m$^2$, formant le matelas, elle-même recouverte d'une couche de non-tissé sous forme de voile cardé classique, formant la couche de surface. Tout autre type de matériau absorbant peut être utilisé pour le matelas, tel que la mousse de polyuréthane ou la mousse de sphaigne traitée.

Le procédé en général, que ce soit par couture, tuftage ou aiguilletage, en l'occurrence l'aiguilletage mécanique, permet de supprimer l'étape d'assemblage des différents matériaux du pré-assemblage formant l'article d'hygiène. Une simple superposition de la couche de surface, du matelas absorbant et de la couche fibreuse externe est suffisante. En effet, la liaison entre les différentes couches de matériau est obtenue directement par l'étape de formation des capteurs sous forme de faisceau d'éléments fibreux traversant les différentes couches de matériaux.

L'avantage d'un tel procédé est la combinaison de la liaison des différents matériaux entre eux et la formation de capteurs par des éléments fibreux entraînés à travers les différents matériaux.

Un scellage de contour est ensuite de préférence réalisé entre la couche de surface et la couche imperméable disposée sous le pré-assemblage pour consolider l'ensemble de l'article et envelopper de manière plus étanche le matelas.

On a réalisé des essais sur des articles munis de capteurs du type bouclette, obtenus par couture.

L'efficacité des capteurs et plus particulièrement le drainage, ont été quantifiés par la mesure de la vitesse d'acquisition en liquide d'une bouclette.

On a préparé des échantillons selon la méthode qui suit. Dans un premier temps, on fabrique le matelas absorbant. On forme une nappe comprenant 80 % de fibres cellulosiques et 20 % de fibres synthétiques thermofusibles, par voie pneumatique, puis on transporte cette nappe déposée au préalable sur une feuille d'ouate de cellulose dans un four de thermoliaison A la sortie du four, une seconde feuille d'ouate de cellulose est placée sur la face supérieure de la nappe. L'ensemble passe ensuite dans une calandre chauffée de manière à lier cette dernière feuille à la nappe.

On prélève dans l'ensemble obtenu, des échantillons d'environ 4 x 4 cm$^2$ , d'un poids d'environ 1 g et d'une épaisseur d'environ 0,60 cm. On dépose à la surface de ces échantillons une couche de surface constituée d'un non-tissé de type "meltblown" commercialisé par la société COROVIN. Ce nontissé a un grammage de 20 g/m$^2$, est perméable à la vapeur d'eau et imperméable aux liquides.

On forme ensuite des capteurs, ici des bouclettes, de manière manuelle, par couture. On utilise un fil multibrins en polyamide 6,6 de 1260 dtex et comprenant 68 brins, commercialisé par RHONE-POULENC. Au moyen d'une aiguille à coudre modifiée, on forme une bouclette dont les deux extrémités du fil multibrins composant la bouclette, traversent le pré-assemblage forme du matelas absorbant et de la couche de surface, en un seul point. De ce fait, les brins sont resserrés au passage du matelas absorbant, formant ainsi le gradient de capillarité, les canaux capillaires étant constitués à l'intérieur d'une bouclette par l'espace entre les brins.

Pour un même échantillon, on peut disposer une ou plusieurs bouclettes selon un motif précis.

On prépare également des échantillons témoins en implantant l'aiguille à coudre selon le même motif que celui-ci prévu pour les échantillons munis de bouclettes, mais sans utiliser de fil.

On a observé aussi bien sur une bouclette laissée intacte, que sur une bouclette dont le sommet avait été coupée, un excellent drainage d'une goutte de liquide synthétique se rapprochant de la formule du sang humain, la goutte étant déposée sur l'extrémité de la bouclette intacte ou ouverte. En effet, dans les deux cas, le liquide est conduit à la base de la bouclette et disparaît dans le matelas absorbant, de manière très rapide et surprenante.

On a préparé cinq échantillons ayant chacun de 1 à 5 bouclettes suivant un motif particulier d'implantation de bouclettes, représenté ci-dessous :

Les bouclettes sont gardées intactes et ont une hauteur d'environ 1 cm.

Cinq échantillons témoins ont été préparés avec la même implantation mais sans fil.

La mesure est ensuite réalisée selon la méthode suivante.

Pour un échantillon donné, les bouclettes sont enfermées dans un tube de diamètre interne de 20 mm, qui est posé sur l'échantillon. On injecte 2 ml de liquide synthétique simulant le flux menstruel, dans le tube en 1seconde 2/10. On déclenche un chronomètre en même temps que l'injection et on l'arrête lorsque toutes les bouclettes sont visuellement "sèches" ou "vides". On mesure ainsi le temps d'acquisition.

On mesure également la quantité de liquide restant en surface de l'échantillon, en posant immédiatement après l'arrêt du chronomètre, deux plis de papier buvard WHATMANN n° 4, de 9 cm sur 14 cm, d'un poids sec moyen de 2,37 g, surmontés d'une masse de 500 g, d'un diamètre de 38 mm.

Or, la quantité de liquide restant en surface de l'échantillon est d'environ 1,6 pour cent en poids de la quantité injectée, quel que soit le nombre de bouclettes sur l'échantillon.

On a effectué la même mesure sur les échantillons témoins. Mais celle-ci est impossible car le liquide ne pénètre pas dans l'échantillon témoin, le temps d'acquisition restant supérieur à 5 minutes, quel que soit le motif d'implantation.

Les résultats des mesures du temps d'acquisition sont rapportés sur la courbe représentée en figure 11 où le temps d'acquisition est fonction du nombre de bouclettes.

Comme on peut l'observer sur la courbe, le temps d'acquisition diminue avec le nombre de bouclettes implantées. Il existe bien sûr une limite supérieure du nombre de bouclettes au delà de laquelle le liquide n'est plus capté par les bouclettes qui forment une couverture trop dense sur laquelle ruisselle le liquide.

Le temps d'acquisition très long pour les témoins n'a pas été rapporté sur la courbe.

Le temps d'acquisition inversement proportionnel au nombre de bouclettes peut se traduire par une loi du type :

$$T = \frac{(t1)}{N}.k + to$$

dans laquelle :

-   t1 est le temps d'acquisition d'une bouclette,
-   N est le nombre de bouclettes,
-   k est le coefficient dépendant de la nature de la couche de surface $0 \le k \le 1$,
    k tendant vers 0 pour un non tissé très perméable et
    k tendant vers 1 pour un non-tissé très hydrophobe, et
-   to est le temps d'écoulement des 2 ml de liquide dans le tube, c'est-à-dire ici 1 seconde 2/10.

Les articles d'hygiène munis de capteurs selon l'invention présentent un drainage exceptionnel.

Cette fonction de drainage combinée à celle de capture des fluides directement au contact du corps, font de cet article un produit très performant.

## Revendications

1.  Article d'hygiène féminine externe (1) tel qu'une serviette périodique, du type comprenant un matelas absorbant les fluides, une couche de surface (4) située sur ledit matelas absorbant (3) et une couche imperméable (5) située sous ledit matelas (3) et destinée à être au contact d'un sous-vêtement, caractérisé en ce que ledit article (1) comprend au moins un capteur de fluides (2) dont l'extrémité libre supérieure (9) est à une distance d de ladite couche de surface (4) et est destinée à être en contact du corps et dont l'extrémité inférieure est située en dessous de la face intérieure (11) dudit matelas (3), en ce que ledit capteur (2) est formé d'un faisceau comprenant au moins un élément fibreux multibrins, la partie (7) dudit capteur (2) située au-dessus de ladite couche de surface (4) ayant une forme évasée convergente.

2. Article d'hygiène selon la revendication 1, caractérisé en ce que ledit capteur (2) est muni de canaux capillaires (12) souples et permanents, formés par les espaces entre les brins dudit faisceau constituant le capteur.

3. Article d'hygiène selon la revendication 1 ou 2, caractérisé en ce que ledit faisceau est une bouclette ouverte ou fermée comprenant au moins un fil multibrins.

4. Article d'hygiène selon l'une des revendications précédentes, caractérisé en ce que d varie entre environ 3 millimètres et environ 20 millimètres et de préférence entre environ 5 et environ 10 millimètres.

5. Article d'hygiène selon l'une des revendications précédentes, caractérisé en ce que chaque élément fibreux dudit faisceau formant le capteur comprend entre environ 2 et environ 200 brins et de préférence entre environ 30 et environ 100 brins.

6. Article d'hygiène selon la revendication 5, caractérisé en ce que chaque brin unitaire a un titre d'au moins 6 dtex et plus préférentiellement d'au moins 17 dtex.

7. Article d'hygiène selon l'une des revendications 3 à 6, caractérisé en ce que ledit élément fibreux multibrins a un titre variant dans l'intervalle d'environ 180 dtex à environ 5000 dtex.

8. Article d'hygiène selon les revendications 5 et 6, caractérisé en ce que chaque brin a un titre d'au moins 17 dtex et chaque élément fibreux comprend au moins 30 brins.

9. Article d'hygiène selon l'une des revendications précédentes, caractérisé en ce que l'élément fibreux précité est d'origine naturelle telle que le coton, artificielle telle que la viscose ou d'origine synthétique telle que le polyamide, le polyester, le polypropylène ou le polyéthylène.

10. Article d'hygiène selon l'une des revendications précédentes, caractérisé en ce que les fibres sont de préférence hydrophiles ou rendues hydrophiles.

11. Article d'hygiène selon l'une des revendications 3 à 10, caractérisé en ce que la surface totale de l'article d'hygiène est recouverte de bouclettes, la hauteur des bouclettes variant proportionnellement à la distance séparant le corps de la couche de surface précitée.

12. Procédé pour fabriquer un article d'hygiène selon l'une des revendications précédentes, caractérisé en ce qu'il consiste à :

   a) former un pré-assemblage du type comprenant un matelas absorbant les fluides et une couche de surface disposée sur ledit matelas ;
   b) disposer une couche externe fibreuse sous ou sur ledit pré-assemblage ;
   c) former à partir de ladite couche fibreuse, au moins un faisceau comprenant au moins un élément fibreux multibrins dont l'extrémité inférieure est fixée au niveau de la face inférieure du matelas, dont la partie externe située au-dessus de la couche de surface est libre et de forme évasée convergente vers ladite couche de surface et dont l'extrémité supérieure est destinée à être au contact du corps ; et
   d) fixer une couche imperméable sous l'article d'hygiène obtenu en c).

13. Procédé selon la revendication 12, caractérisé en ce que la couche externe fibreuse est disposée sur l'assemblage précité et est formée d'au moins un fil multibrins continu ou discontinu et en ce que ledit fil multibrins est piqué par couture à travers le pré-assemblage précité de manière à former au moins un faisceau sous forme de bouclette formée de fil multibrins.

14. Procédé selon la revendication 12, caractérisé en ce que la couche externe fibreuse est disposée sous l'assemblage précité et est formée d'au moins un fil multibrins continu ou discontinu et en ce que ledit fil multibrins est piqué par une technique connue de tuftage à travers l'assemblage précité de manière à former au moins un faisceau sous forme de bouclette formée de fils multibrins.

**15.** Procédé selon la revendication 14, caractérisé en ce que la couche de surface est de préférence un matériau textile nontissé.

**16.** Procédé selon la revendication 13, 14 ou 15, caractérisé en ce que ledit faisceau sous forme de bouclette est coupé.

**17.** Procédé selon la revendication 12, caractérisé en ce que ladite couche externe fibreuse est constituée d'une nappe ou voile de fibres et est disposée sous le pré-assemblage précité et en ce que l'on fait passer des éléments fibreux issus de ladite couche externe à travers le pré-assemblage par une technique connue d'aiguilletage pour former un faisceau d'élément multibrins, dont la partie à la surface de l'article d'hygiène est de forme évasée et convergente.

**18.** Procédé selon la revendication 17, caractérisé en ce que l'aiguilletage précité est un aiguilletage de type mécanique.

## Claims

**1.** External feminine hygiene article (1) such as a sanitary towel, of the type which comprises a pad which absorbs the fluids, a surface layer (4) which is disposed on the said absorbent pad (3), and an impermeable layer (5) which is disposed beneath the said pad (3), and is designed to be in contact with an under-garment, characterised in that the said article (1) comprises at least one fluid collector (2), the upper free end (9) of which is at a distance d from the said surface layer (4), and is designed to be in contact with the body, and the lower end of which is disposed below the lower surface (11) of the said pad (3), and in that the said collector (2) consists of a bundle comprising at least one multi-strand fibrous element, the part (7) of the said collector (2) which is disposed above the said surface layer (4) having a converging flared shape.

**2.** Hygiene article according to claim 1, characterised in that the said collector (2) is provided with flexible, permanent capillary channels (12), formed by the spaces between the strands of the said bundle which constitutes the collector.

**3.** Hygiene article according to claim 1 or claim 2, characterised in that the said bundle is an open or closed loop, comprising at least one multi-strand thread.

**4.** Hygiene article according to any one of the preceding claims, characterised in that d varies between approximately 3 mm and approximately 20 mm, and preferably between approximately 5 and approximately 10 mm.

**5.** Hygiene article according to any one of the preceding claims, characterised in that each fibrous element of the said bundle which forms the collector comprises between approximately 2 and approximately 200 strands, and preferably between approximately 30 and approximately 100 strands.

**6.** Hygiene article according to claim 5, characterised in that each individual strand has a yarn count of at least 6 dtex, and more preferably at least 17 dtex.

**7.** Hygiene article according to any one of claims 3 to 6, characterised in that the said multi-strand fibrous element has a yarn count which varies in the interval of approximately 180 dtex to approximately 5000 dtex.

**8.** Hygiene article according to claim 5 and claim 6, characterised in that each strand has a yarn count of at least 17 dtex, and each fibrous element comprises at least 30 strands.

**9.** Hygiene article according to any one of the preceding claims, characterised in that the aforementioned fibrous element is of natural origin such as cotton, artificial origin such as viscose, or synthetic origin such as polyamide, polyester, polypropylene or polyethylene.

**10.** Hygiene article according to any one of the preceding claims, characterised in that the fibres are preferably hydrophile, or are rendered hydrophile.

**11.** Hygiene article according to any one of claims 3 to 10, characterised in that the entire surface of the hygiene article is covered with loops, the height of the loops varying in proportion with the distance which separates the body from the aforementioned surface layer.

**12.** Method for production of a hygiene article according to any one of the preceding claims, characterised in that it consists of:

a) forming a pre-assembly of the type which comprises a pad which absorbs the fluids, and a surface layer which is disposed on the said pad;
b) placing an outer fibrous layer beneath or on the said pre-assembly;
c) forming, starting from the said fibrous layer, at least one bundle, comprising at least one multi-strand fibrous element, the lower end of which is secured at the level of the lower surface of the pad, the outer part of which, which is disposed above the said surface layer, is free, and has a flared shape which converges towards the said surface layer, and the upper end of which is designed to be in contact with the body; and
d) securing an impermeable layer beneath the hygiene article obtained in c).

**13.** Method according to claim 12, characterised in that the outer fibrous layer is disposed on the aforementioned pre-assembly, and consists of at least one continuous or discontinuous multi-strand thread, and in that the said multi-strand thread is stitched through the aforementioned pre-assembly, such as to form at least one bundle in the form of a loop consisting of multi-strand threads.

**14.** Method according to claim 12, characterised in that the outer fibrous layer is disposed beneath the aforementioned assembly, and consists of at least one continuous or discontinuous multi-strand thread, and in that the said multi-strand thread is sewn by means of a known tufting technique through the aforementioned assembly, such as to form at least one bundle in the form of a loop consisting of multi-strand threads.

**15.** Method according to claim 14, characterised in that the surface layer is preferably a non-woven textile material.

**16.** Method according to claim 13, claim 14 or claim 15, characterised in that the said bundle in the form of a loop is cut.

**17.** Method according to claim 12, characterised in that the said outer fibrous layer consists of a fleece or sheeting of fibres, and is disposed beneath the aforementioned pre-assembly, and in that fibrous elements obtained from the said outer layer are passed through the pre-assembly by means of a known tufting technique, in order to form a bundle of multi-strand elements, the part of which that is on the surface of the hygiene article has a flared, converging shape.

**18.** Method according to claim 17, characterised in that the aforementioned tufting is of a mechanical type.

## Patentansprüche

**1.** Hygieneartikel (1) für die externe weibliche Hygiene, insbesondere Monatsbinde, mit einer flüssigkeitsabsorbierenden Einlage, einer Oberflächenschicht (4), die auf der absorbierenden Einlage (3) angordnet ist, und einer undurchlässigen Schicht (5), die unter der Einlage (3) angeordnet ist und mit der Unterwäsche in Berührung gelangt, dadurch gekennzeichnet, daß der Artikel (1) mindestens einen Flüssigkeits-Aufnehmer (2) aufweist, dessen oberes freies Ende (9) einen Abstand d von der Oberflächenschicht (4) hat und mit dem Körper in Berührung gelangt und dessen unteres Ende unterhalb der Unterseite (11) der Einlage (3) angeordnet ist, und daß der Aufnehmer (2) die Form eines Büschels aus mindestens einem Multifilament-Faserelement hat, wobei der oberhalb der Oberflächenschicht (4) angeordnete Abschnitt (7) des Aufnehmers (2) eine aufgeweitete konvergierende Form hat.

**2.** Hygieneartikel nach Anspruch 1, dadurch gekennzeichnet, daß der Aufnehmer (2) mit biegsamen und permanenten kapillaren Kanälen (12) versehen ist, die durch die Zwischenräume zwischen den Filamenten des den Aufnehmer bildenden Büschels gebildet werden.

**3.** Hygieneartikel nach Anspruch 1 oder 2, dadurch gekennzeichnet, daß das Büschel eine offene oder geschlossene Schlinge aus mindestens einer Multifilamentfaser ist.

**4.** Hygieneartikel nach einem der vorhergehenden Ansprüche, dadurch gekennzeichnet, daß d zwischen ungefähr 3 mm und ungefähr 20 mm und vorzugsweise zwischen ungefähr 5 mm und 10 mm variiert.

5. Hygieneartikel nach einem der vorhergehenden Ansprüche, dadurch gekennzichnet, daß jedes Faserelement des den Aufnehmer bildenden Büschels zwischen ungefähr 2 und ungefähr 200 Filamenten und vorzugsweise zwischen ungefähr 30 und ungefähr 100 Filamenten aufweist.

6. Hygieneartikel nach Anspruch 5, dadurch gekennzeichnet, daß jedes einzelne Filament einen Titer von mindestens 6 dtex und vorzugsweise von mindestens 17 dtex hat.

7. Hygieneartikel nach einem der Ansprüche 3 bis 6, dadurch gekennzeichnet, daß das Multifilament-Faserelement einen Titer hat, der in einem Bereich von ungefähr 180 dtex bis ungefähr 5000 dtex variiert.

8. Hygieneartikel nach den Ansprüchen 5 und 6, dadurch gekennzeichnet, daß jedes Filament einen Titer von mindstens 17dtex hat und jedes Faserelement mindestens 30 Filamente aufweist.

9. Hygieneartikel nach einem der vorhergehenden Ansprüche, dadurch gekennzeichnet, daß das besagte Faserelement aus einem Material auf natürlicher Basis, z.B. Baumwolle, künstlicher Basis, z.B. Viskose, oder synthetischer Basis, z.B. Polyamid, Polyester, Polypropylen oder Polyäthylen, besteht.

10. Hygieneartikel nach einem der vorhergehenden Ansprüche, dadurch gekennzeichnet, daß die Fasern vorzugsweise hydrophil sind oder hydrophil gemacht werden.

11. Hygieneartikel nach einem der Ansprüche 3 bis 10, dadurch gekennzeichnet, daß die gesamte Oberfläche des Hygieneartikels mit Schlingen bedeckt ist, wobei die Höhe der Schlingen proportional zu dem Abstand zwischen dem Körper und der Oberflächenschicht ist.

12. Verfahren zum Herstellen eines Hygieneartikels nach einem der vorhergehenden Ansprüche, dadurch gekennzeichnet, daß es aus den folgenden Schritten besteht:

a) Herstellen eines Zwischengebildes aus einer flüssigkeitsabsorbierenden Einlage und einer auf der Einlage angeordneten Oberflächenschicht;
b) Anordnen einer äußeren Faserschicht unter oder auf dem Zwischengebilde;
c) Versehen der Faserschicht mit mindestens einem Büschel aus mindestens einem Multifilament-Faserelement, dessen unteres Ende im Bereich der Unterseite der Einlage befestigt ist, dessen äußerer Abschnitt, der oberhalb der Oberflächenschicht liegt, frei ist und eine aufgeweitete konvergente Form in Richtung auf die Oberflächenschicht hat und dessen oberes Ende mit dem Körper in Berührung gelangt, und
d) Befestigen einer undurchlässigen Schicht unterhalb des durch den Schritt c) erhaltenen Hygieneartikels.

13. Verfahren nach Anspruch 12, dadurch gekennzeichnet, daß die äußere Faserschicht an dem besagten Zwischengebilde angeordnet wird und aus mindestens einem kontinuierlichen oder diskontinuierlichen Multifilamentfaden besteht, und daß der Multifilamentfaden maschinell in einem Nähvorgang durch das Zwischengebilde so gezogen wird, daß mindestens ein Büschel in Form einer von dem Multifilamentfaden gebildeten Schlinge entsteht.

14. Verfahren nach Anspruch 12, dadurch gekennzeichnet, daß die äußere Faserchicht unter dem Zwischengebilde angeordnet und mit mindestens einem kontinuierlichen oder diskontinuierlichen Multifilamentfaden versehen wird, und daß der Multifilamentfaden durch eine bekannte Tufting-Technik durch das Zwischengebilde so gezogen wird, daß mindestens ein Büschel in Form einer von Multifilamentfäden gebildeten Schlinge entsteht.

15. Verfahren nach Anspruch 14, dadurch gekennzeichnet, daß die Oberflächenschicht vorzugsweise aus einem textilen Vliesmaterial besteht.

16. Verfahren nach Anspruch 13, 14 oder 15, dadurch gekennzeichnet, daß das Büschel in Form einer Schlinge aufgeschnitten wird.

17. Verfahren nach Anspruch 12, dadurch gekennzeichnet, daß die äußere Faserschicht von einem Faserband gebildet und unterhalb des Zwischengebildes angeordnet wird und daß man die aus der Außenschicht ragenden Faserelemente durch das Zwischengebilde mittels einer bekannten Nadeltechnik zieht, um ein Multifilament-Büschel zu bilden, dessen auf der Oberfläche des Hygieneartikels liegender Abschnitt eine aufgeweitete und konvergente Form hat.

**18.** Verfahren nach Anspruch 17, dadurch gekennzeichnet, daß es sich bei der Nadeltechnik um eine mechanische Nadelung handelt.

FIG.1

FIG.2

FIG.3

FIG. 4a

FIG. 4b

FIG.5

FIG.10

FIG. 6a

FIG. 6b

FIG. 6c

FIG. 6d

FIG. 6e

FIG.7

FIG.8a

FIG.8b

FIG.8c

FIG.9a

FIG.9b

FIG.9c

**FIG.11**